(19) Europäisches Patentamt

European Patent Office

Office européen des brevets

(11) **EP 0 748 797 B2**

(12) **NOUVEAU FASCICULE DE BREVET EUROPEEN**

(45) Date de publication et mention de la décision concernant l'opposition:
**08.06.2005 Bulletin 2005/23**

(51) Int Cl.$^7$: **C07D 201/08**

(45) Mention de la délivrance du brevet:
**09.01.2002 Bulletin 2002/02**

(21) Numéro de dépôt: **96420199.0**

(22) Date de dépôt: **07.06.1996**

(54) **Procédé de préparation de caprolactame à partir d'aminocapronitrile**

Verfahren zur Herstellung von Caprolactam aus Aminocapronitril

Process for the preparation of caprolactam from aminocapronitrile

(84) Etats contractants désignés:
**BE DE ES FR GB IT NL**

(30) Priorité: **16.06.1995 FR 9507446**

(43) Date de publication de la demande:
**18.12.1996 Bulletin 1996/51**

(73) Titulaire: **Rhodia Polyamide Intermediates**
**69192 Saint-Fons (FR)**

(72) Inventeurs:
• **Gilbert, Laurent**
**75015 Paris (FR)**
• **Laurain, Nathalie**
**69003 Lyon (FR)**
• **Leconte, Philippe**
**69330 Meyzieu (FR)**

• **Nedez, Christophe**
**92600 Asnieres sur Seine (FR)**

(74) Mandataire: **Esson, Jean-Pierre et al**
**Rhodia Services,**
**Direction de la Propriété Industrielle,**
**Centre de Recherches de Lyon**
**BP 62**
**85, avenue des Frères Perret**
**69192 Saint-Fons Cédex (FR)**

(56) Documents cités:

| EP-A- 0 150 295 | WO-A-93/14064 |
| WO-A-93/16984 | WO-A-96/36601 |
| DE-A- 4 319 134 | DE-A- 4 339 648 |
| FR-A- 2 029 540 | US-A- 2 208 598 |
| US-A- 2 301 964 | US-A- 2 357 484 |
| US-A- 2 762 835 | US-A- 4 625 023 |
| US-A- 4 628 085 | US-A- 5 151 543 |

EP 0 748 797 B2

**Description**

La présente invention concerne un procédé de préparation de lactames à partir de dinitriles.

Les lactames aliphatiques, tels que notamment l'epsilon-caprolactame, sont des composés de base pour la préparation des polyamides (polyamide-6 à partir du caprolactame).

Industriellement, le caprolactame est préparé à partir de la cyclohexanone, qui est transformée en son oxime. L'oxime est ensuite transformée en caprolactame en présence d'un acide fort, tel que l'acide sulfurique, dont il faut ensuite neutraliser l'excès. Un tel procédé présente l'inconvénient majeur de générer de très grosses quantités de sulfate d'ammonium, jusqu'à plusieurs tonnes par tonne de caprolactame préparé.

Un autre moyen envisagé de préparer ces lactames consiste à effectuer une hydrogénation partielle de dinitriles en aminonitriles correspondants, plus particulièrement de l'adiponitrile en aminocapronitrile, puis à purifier soigneusement l'aminonitrile, afin d'éliminer toutes les impuretés, telles que diverses imines ou amines formées lors de l'hydrogénation ou encore la diamine et enfin à polymériser en polyamide l'aminonitrile ainsi purifié.

Ce dernier procédé qui paraît séduisant à première vue, nécessite impérativement une purification très poussée de l'aminonitrile. En effet, la polymérisation n'est envisageable que si aminonitrile est exempt de diamine, et surtout des différents sous-produits imines ou amines qui limitent le degré de polymérisation et provoquent l'apparition de couleurs et de ramifications. Cependant la proximité des propriétés de l'aminonitrile et des imines ou amines à éliminer et surtout l'existence d'équilibres entre différentes formes de sous-produits sont telles que la séparation n'est pas aisée et qu'il est nécessaire d'ajouter un ou plusieurs composés destinés à transformer les imines avant de les éliminer. Ainsi le brevet WO-A-9316984 propose d'ajouter au mélange à traiter jusqu'à 10 % en poids d'un composé carbonylé, tel qu'un aldéhyde ou une cétone. Le brevet WO-A-9314064 enseigne d'introduire un composé méthylénique, tel que malonitrile, dicyclopentadiène, cyclopentadiène, nitrométhane, nitroéthane, indène, au mélange réactionnel avant de le distiller. Cette addition de composés à un mélange déjà complexe n'est pas de nature à simplifier le procédé.

Par ailleurs de nombreux documents décrivent la fabrication de lactames à partir d'aminonitriles par mise en oeuvre d'une réaction d'hydrolyse cyclisante. Ainsi, les brevets EP0150295, FR2029540 DE4319134 et DE4339648 décrivent une telle réaction mise en oeuvre avec un aminonitrile pur. Enfin, les brevets US4628085 et US4625023 décrivent la mise en oeuvre d'une telle réaction d'hydrolyse cyclisante sur un mélange aminonitrile / dinitrile, le dinitrile n'étant pas transformé au cours de la réaction. En conséquence, ce procédé requiert la séparation du ditrile et du lactame formé. De plus, ces documents précisent que des composés imines se forment au cours de la réaction d'hydrolyse.

La présente invention consiste en un procédé de préparation de lactame, enchaînant deux étapes, l'une d'hémihydrogénation de dinitrile en aminonitrile, l'autre d'hydrolyse cyclisante de l'aminonitrile après seulement une opération simple de purification.

Plus précisément l'invention consiste en un procédé de préparation de lactame, caractérisé en ce qu'il comprend les étapes successives suivantes :

- hémihydrogénation d'un dinitrile aliphatique en aminonitrile par l'hydrogène et en présence d'un catalyseur et d'une base minérale forte,
- extraction de l'aminonitrile du milieu réactionnel par distillation pour récupérer un aminonitrile présentant une teneur en dinitrile variant de 0,005% à 5% en poids et une teneur en sous-produits à fonction imine ou amine variant de 0,2% à 5% en poids,
- hydrolyser l'aminonitrile distillé en phase vapeur avec de l'eau en présence ou non d'un catalyseur pour produire un lactame.

Par distillation on peut obtenir de l'aminonitrile ayant une teneur pondérale en dinitrile de 0,0050 %, mais cette faible teneur s'obtient au prix de la quantité d'aminonitrile disponible, car il faut séparer des fractions de distillation intermédiaire que l'on peut recycler dans une nouvelle opération d'hémihydrogénation. Aussi préfère-t-on généralement mettre en oeuvre dans l'étape d'hydrolyse cyclisante un aminonitrile contenant de 0,005 % à 5 % en poids de dinitrile.

Il est généralement difficile par distillation d'avoir dans l'aminonitrile une teneur globale dans les autres sous-produits inférieure à 0,2 %, bien que cette valeur ne constitue pas une limite inférieure critique pour le procédé de l'invention.

De manière générale, on met en oeuvre dans l'étape d'hydrolyse cyclisante un aminonitrile contenant de 0,2 % à 5 % en poids de sous-produits à fonction imine ou amine.

La diamine correspondant au dinitrile mis en oeuvre n'est pas gênante pour l'étape d'hydrolyse cyclisante. Elle n'est pas considérée dans le présent texte comme un sous-produit à fonction amine mentionné précédemment.

Les dinitriles aliphatiques qui peuvent être mis en oeuvre dans la première étape du procédé de l'invention sont plus particulièrement les dinitriles de formule générale (I):

$$NC\!-\!R\!-\!CN \qquad\qquad (I)$$

dans laquelle R représente un groupement alkylène ou alcénylène, linéaire ou ramifié, ayant de 1 à 12 atomes de carbone.

De préférence, on met en oeuvre des dinitriles de formule (I) dans laquelle R représente un radical alkylène, linéaire ou ramifié ayant de 1 à 6 atomes de carbone.

A titre d'exemples de tels dinitriles, on peut citer notamment l'adiponitrile, le méthylglutaronitrile, l'éthylsuccinonitrile, le malononitrile, le succinonitrile et le glutaronitrile et leurs mélanges, notamment les mélanges d'adiponitrile et/ou de méthylglutaronitrile et/ou d'éthylsuccinonitrile susceptibles de provenir d'un même procédé de synthèse de l'adiponitrile.

En pratique, le cas où R = $(CH_2)_4$ sera le plus fréquent car cela correspond à la mise en oeuvre de l'adiponitrile (ADN) dans le présent procédé.

L'hémihydrogénation du dinitrile en aminonitrile correspondant, à l'aide d'hydrogène, est effectuée en général en présence d'un catalyseur à base de nickel de Raney, de cobalt de Raney, de nickel de Raney ou de cobalt de Raney comportant un élément dopant choisi parmi les éléments des groupes IVb, Vlb, Vllb et VIII de la classification périodique des éléments telle que publiée dans Handbook of Chemistry and Physics, 51st Edition (1970-1971) et d'une base minérale forte dérivant d'un métal alcalin ou alcalino-terreux.

Le milieu initial d'hydrogénation comporte au moins un solvant apte à solubiliser au moins partiellement le dinitrile, car l'hydrogénation s'opère mieux lorsque ledit dinitrile est dissous. Ce milieu solvant peut être constitué par de l'eau à raison d'au moins 0,5 % en poids par rapport à la totalité des composés liquides du milieu réactionnel.

En complément ou en subtitution de l'eau, le milieu réactionnel peut contenir au moins un autre solvant, tel qu'un alcool et/ou un amide et/ou une amine et/ou de l'ammoniac. Les alcools qui conviennent plus particulièrement sont les alcanols comme le méthanol, l'éthanol, le propanol-1, le propanol-2 et le butanol-1, les diols comme l'éthylèneglycol et le propylèneglycol, les polyols ou les mélanges desdits alcools. Dans le cas où le solvant est un amide, on peut faire appel notamment au diméthylformamide et au diméthylacétamide. Parmi les amines qui peuvent être utilisées comme solvant, on peut mettre en oeuvre par exemple la diamine ou l'aminonitrile correspondant au dinitrile que l'on hydrogène.

Lorsqu'il est employé avec l'eau, le solvant représente en poids de deux à quatre fois le poids de l'eau.

Selon une variante préférée de l'étape d'hémihydrogénation du dinitrile, le milieu réactionnel initial comprend de la diamine et/ou de l'aminonitrile susceptibles de se former à partir du dinitrile à hydrogéner ainsi que du dinitrile non transformé à raison pour l'ensemble de ces trois composés de 80 % à 99,5 %.

Le taux de transformation du dinitrile est de préférence d'au moins 70 %.

La base minérale forte est généralement constituée par les hydroxydes, les carbonates et les alcanolates de métal alcalin ou de métal alcalino-terreux. Elle est choisie de préférence parmi les hydroxydes, les carbonates et les alcanolates de métal alcalin. De façon privilégiée, la base minérale forte mise en oeuvre est choisie parmi les composés suivants : LiOH, NaOH, KOH, RbOH, CsOH et leur mélanges.

En pratique, on utilise le plus souvent NaOH et KOH, pour un bon compromis performance-prix, bien que RbOH et CsOH puissent donner de très bons résultats.

Le milieu réactionnel a une composition variant selon le type de mise en oeuvre du procédé.

En effet si le procédé est mise en oeuvre en mode discontinu comme c'est notamment le cas dans les réalisations de laboratoire ou de petites fabrications intermittentes, le milieu réactionnel initial s'enrichira progressivement en aminonitrile et, en moindre proportion, en diamine, tandis que la concentration en dinitrile pourra soit décroître si l'on charge la totalité ou la majeure partie dudit dinitrile dès le début de l'hémihydrogénation, soit demeurer relativement constante si le dinitrile est introduit progressivement au cours de la réaction.

Par contre, si le procédé est conduit en mode continu, la composition du milieu réactionnel en sortie de réacteur atteint des valeurs déterminées par les sélectivités de la réaction.

L'eau est habituellement présente dans une quantité inférieure ou égale à 20 %. Préférentiellement, la teneur en eau du milieu réactionnel est comprise entre 2 % et 15 % en poids par rapport à l'ensemble des constituants liquides dudit milieu.

En fonctionnement continu du procédé de l'invention, la composition moyenne sera déterminée par le rapport des sélectivités respectives en aminonitrile et en diamine et par la vitesse d'introduction du dinitrile.

La quantité de base minérale forte est avantageusement supérieure ou égale à 0,1 mol/kg de catalyseur. De préférence, elle est comprise entre 0,1 mol et 3 mol par kg de catalyseur et plus préférentiellement encore entre 0,3 et 2 mol/kg de catalyseur.

Le catalyseur utilisé dans le procédé peut être un nickel de Raney, un cobalt de Raney, un nickel de Raney ou un cobalt de Raney comportant, outre le nickel ou le cobalt et les quantités résiduelles du métal éliminé de l'alliage d'origine lors de la préparation du catalyseur, c'est-à-dire généralement l'aluminium, un ou plusieurs autres éléments, souvent appelés dopants, tels que par exemple le chrome, le titane, le molybdène, le tungstène, le fer, le zinc. Parmi ces éléments dopants le chrome et/ou le fer et/ou le titane sont considérés comme les plus avantageux. Ces dopants représentent habituellement, en poids par poids de nickel, de 0 % à 15 % et de préférence de 0 % à 10 %.

La quantité de catalyseur mise en oeuvre peut varier très largement en fonction notamment du mode de fonctionnement adopté ou des conditions réactionnelles choisies. Ainsi, si l'on introduit progressivement le dinitrile dans le milieu réactionnel, le rapport pondéral catalyseur/dinitrile à hydrogéner sera beaucoup plus élevé que si tout le dinitrile est mis en oeuvre dès le début de la réaction. A titre indicatif, on peut utiliser de 0,5 % à 50 % en poids de catalyseur par rapport au poids total du milieu réactionnel et le plus souvent de 1 % à 35 %.

Pour un catalyseur donné et pour un taux de transformation donné du dinitrile, le rendement en aminonitrile passe par un maximum déterminé par le rapport base/Ni ou base/Co.

L'optimum de rendement en aminonitrile, à taux de transformation du dinitrile constant, dépend de la nature et de la teneur en dopant, de la quantité d'eau dans le milieu réactionnel et de la température.

L'étape d'hémihydrogénation du procédé de l'invention est généralement effectuée à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C. On peut sans problème technique opérer à une température inférieure à 20°C, mais cela ne présente pas d'intérêt en raison d'une productivité plus faible de la réaction.

Préalablement, simultanément ou postérieurement au chauffage, l'enceinte réactionnelle est amenée à la pression en hydrogène convenable, c'est-à-dire, en pratique, comprise entre 1bar (0,10 MPa) et 100 bar (10 MPa) et de préférence entre 5 bar (0,5 MPa) et 50 bar (5 MPa).

La durée de la réaction est variable en fonction des conditions réactionnelles et du catalyseur.

Dans un mode de fonctionnement discontinu, elle peut varier de quelques minutes à plusieurs heures.

Dans un mode de fonctionnement continu, qui constitue le mode industriel préférable pour le procédé selon l'invention, la durée n'est évidemment pas un paramètre figeable.

Avant de soumettre l'aminonitrile obtenu dans l'étape d'hémihydrogénation à l'étape d'hydrolyse cyclisante pour former le lactame, il est nécessaire d'éliminer la majeure partie de l'eau et/ou du solvant éventuellement présents, du dinitrile n'ayant pas réagi, de la diamine également formée et des sous-produits de la réaction, tels que les composés de type imine ou amine.

Cette purification peut être commodément réalisée par une opération classique de distillation, de préférence conduite sous une pression inférieure à la pression atmosphérique. L'eau et/ou le solvant distillent tout d'abord, suivis par la diamine formée, par exemple l'hexaméthylènediamine. Ensuite vient l'aminonitrile, par exemple l'amino-6 capronitrile (ACN), tandis que le dinitrile n'ayant pas réagi peut être ou non également séparé par distillation.

Pour son utilisation dans la réaction d'hydrolyse cyclisante, l'aminonitrile obtenu par cette distillation peut contenir, comme cela a ete précisé précédemment, de 0,005% à 5% en poids de dinitrile et de 0,2% à 5% en poids, d'autres sous-produits se formant lors de l'hydrogénation du dinitrile.

Lorsque le dinitrile est l'adiponitrile, ces sous-produits peuvent notamment être de l'hexaméthylène-imine (HMI), de l'aminométhylcyclopentylamine (AMCPA), de l'azacycloheptène (AZCHe), de l'imino-1 cyano-2 cyclopentane (ICCP), du diaminocyclohexane (DCH), de la bis(hexaméthylènetriamine) (BHT), du produit de condensation de l'azacycloheptène avec l'amino-6 capronitrile ((amino-6' hexaméthylène-imino)-6 hexanenitrile) ou, lorsque l'hémihydrogénation est conduite dans un alcool, divers composés de réaction de cet alcool avec des intermédiaires réactionnels.

L'étape d'hydrolyse cyclisante de l'aminonitrile purifié, afin de préparer le lactame correspondant consiste en une réaction en phase vapeur dudit aminonitrile aliphatique de formule générale (II) :

$$N \equiv C - R - CH_2 - NH_2 \tag{II}$$

dans laquelle R représente un radical alkylène ou alcénylène, linéaire ou ramifié, ayant de 1 à 12 atomes de carbone, avec de l'eau, de préférence en présence d'un catalyseur solide.

De préférence, dans la formule (II) de l'aminonitrile, R représente un radical alkylène, linéaire ou ramifié ayant de 1 à 6 atomes de carbone.

Le catalyseur solide peut être de nature très variée. On peut utiliser un tamis moléculaire (composé cristallin à microporosité de 3 à 10 angstroms environ), un tamis moléculaire non-zéolithique, un phosphate métallique ou un oxyde massique acide ou amphotère.

Les tamis moléculaires sont les silicalites et les zéolithes acides.

Par zéolithe, on entend un tectosilicate cristallisé d'origine naturelle ou synthétique dont les cristaux résultent de l'assemblage tridimensionnel d'unités tétraédriques de $SiO_4$ et $TO_4$, T représentant un élément trivalent tel que aluminium, gallium, bore et fer, de préférence l'aluminium. Les zéolithes de type aluminosilicate sont les plus communes.

Les zéolithes présentent au sein du réseau cristallin, un système de cavités reliées entre elles par des canaux d'un diamètre bien défini que l'on appelle les pores.

Les zéolithes peuvent présenter un réseau monodimensionnel, bidimensionnel ou tridimensionnel.

Parmi les zéolithes, on peut utiliser des zéolithes naturelles comme par exemple l'offrétite, la clinoptilotite, l'érionite, la chabazite, la philipsite.

Conviennent aussi tout à fait les zéolithes synthétiques.

Comme exemples de zéolithes synthétiques à réseau monodimensionnel, on peut citer entre autres la zéolithe ZSM-4, la zéolithe L, la zéolithe ZSM-12, la zéolithe ZSM-22, la zéolithe ZSM-23, la zéolithe ZSM-48.

A titre d'exemples de zéolithes à réseau bidimensionnel que l'on utilise préférentiellement, on peut citer la zéolithe bêta, la mordénite, la ferrierite.

En ce qui concerne les zéolithes à réseau tridimensionnel, on peut mentionner plus particulièrement la zéolithe Y, la zéolithe X, la zéolithe ZSM-5, la zéolithe ZSM-11, l'offrétite.

On fait appel de préférence aux zéolithes synthétiques et plus particulièrement à ceux qui sont sous les formes suivantes :

- la mazzite de rapport molaire Si/Al de 3,4
- la zéolithe L de rapport molaire Si/Al de 1,5 à 3,5
- la mordénite de rapport molaire Si/Al de 5 à 15
- la ferrierite de rapport molaire Si/Al de 3 à 10
- l'offrétite de rapport molaire Si/Al de 4 à 8,5
- les zéolithes bêta de rapport molaire Si/Al de 15 à 25
- les zéolithes Y en particulier les zéolithes obtenues après traitement de désalumination (par exemple hydrotraitement, lavage à l'aide d'acide chlorhydrique ou traitement par $SiCl_4$), plus particulièrement les zéolithes US-Y de rapport molaire Si/Al supérieur à 3, de préférence compris entre 6 et 60
- la zéolithe X de type faujasite de rapport molaire Si/Al de 0,7 à 1,5
- les zéolithes ZSM-5 ou silicalite d'aluminium de rapport molaire Si/Al de 10 à 2000
- la zéolithe ZSM-11 de rapport molaire de 5 à 30.

Les zéolithes mises en oeuvre dans le présent procédé sont des produits connus décrits dans la littérature [cf. Atlas of zeolites structure types by W. M. Meier and D. H. Olson, published by the Structure Commission of the International Zeolite Association (1978)].

On peut faire appel aux zéolithes disponibles dans le commerce ou les synthétiser selon les procédés décrits dans la littérature.

On peut se référer à l'Atlas précité et plus particulièrement pour la préparation :

- de la zéolithe L à la publication de Barrer R. M. et al, Z. Kristallogr. 128, pp 352 et suivantes (1969)
- de la zéolithe ZSM-12, au brevet US 3 832 449 et à l'article de Lapierre et al, Zeolites 5, pp 346 et suivantes (1985)
- de la zéolithe ZSM-22 à la publication de Kokotailo G.T. et al, Zeolites 5, pp 349 et suivantes (1985)
- de la zéolithe ZSM-23, au brevet US 4 076 842 et à l'article de Rohrman A.C. et al, Zeolites 5, pp 352 et suivantes (1985)
- de la zéolithe ZSM-48, aux travaux de Schlenker J.L. et al, Zeolites 5, pp 355 et suivantes (1985)
- de la zéolithe bêta, au brevet US 3 308 069 et à l'article de Caullet P. et al, Zeolites 12, pp 240 et suivantes (1992)
- de la mordénite, aux travaux de Itabashi et al, Zeolites 6, pp 30 et suivantes (1986)
- des zéolithes X et Y, respectivement aux brevets US 4 076 842 et US 3 130 007
- de la zéolithe ZSM-5, au brevet US 3 702 886 et à l'article de Shiralkar.V.P. et al, Zeolites 9, pp 363 et suivantes (1989)
- de la zéolithe ZSM-11, aux travaux de Harrison I.D. et al, Zeolites 7, pp 21 et suivantes (1987).

Les zéolithes peuvent être utilisées sous différentes formes : poudre, produits mis en forme tels que granulés (par exemple cylindres ou billes), pastilles, monolithes (blocs en forme de nids d'abeilles) qui sont obenus par extrusion, moulage, compactage ou tout autre type de procédé connu. En pratique, sur le plan industriel, ce sont les formes de granulés, de billes ou de monolithes qui présentent le plus d'avantages tant sur le plan de l'efficacité que sur le plan de la commodité de mise en oeuvre.

L'invention n'exclut pas la présence de liants utilisés lors de la mise en forme de la zéolithe, par exemple des alumines ou des argiles.

Quelle que soit la zéolithe choisie, on fait si nécessaire un traitement qui la rend acide. On fait appel à cet effet aux traitements classiques.

Ainsi, on peut échanger les cations alcalins en soumettant la zéolithe à traitement réalisé avec de l'ammoniaque, conduisant ainsi à un échange de cation alcalin par un ion ammonium, puis à calciner la zéolithe échangée afin de décomposer thermiquement le cation ammonium et le remplacer par un ion H+.

La quantité d'ammoniaque à mettre en oeuvre est au moins égale à la quantité nécessaire pour échanger tous les cations alcalins par des ions ammonium. On met donc en jeu au moins de $10^{-5}$ à $5.10^{-3}$ mole d'ammoniaque par gramme de zéolithe.

Parmi les silicalites, on utilise plus particulièrement la silicalite de type 1 de structure analogue à ZSM-5, la silicalite de type 2 de structure analogue à ZSM-11 et la silicalite béta.

Le terme "tamis moléculaire non-zéolithique" ou NZMS inclut dans le présent texte les tamis moléculaires SAPO décrits dans le brevet US 4 440 871, les tamis moléculaires ELAPSO décrits dans le brevet EP-A-0 159 624 et certains aluminophosphates cristallins, métalloaluminophosphates (MeAPO), ferroaluminophosphates (FeAPO), titanoaluminophosphates (TAPO) décrits dans les brevets indiqués ci-après. Les aluminophosphates cristallins sont décrits dans le brevet US 4 310 440 ; les métalloaluminophosphates cristallins MeAPO, Me représentant au moins un métal choisi parmi Mg, Mn, Co et Zn, sont décrits dans le brevet US 4 567 029 ; les ferroaluminophosphates cristallins FeAPO sont décrits dans le brevet US 4 554 143; les titanoaluminophosphates TAPO sont décrits dans le brevet US 4 500 651 ; d'autres tamis moléculaires non-zéolithiques ELAPO sont décrits dans les brevets EP-A-0 158 976 et EP-A-0 158 349.

Le phosphate métallique peut plus précisément être un phosphate métallique de formule générale (III) :

$$(PO_4)_n \, H_h \, M, \, (Imp)_p \qquad\qquad (III)$$

dans laquelle :

- M représente un élément divalent, trivalent, tétravalent ou pentavalent choisi dans les groupes 2a, 3b, 4b ,5b, 6b, 7b, 8, 2b, 3a, 4a et 5a de la classification périodique des éléments ou un mélange de plusieurs de ces éléments ou M=O lorsque M représente certains éléments pentavalents,
- Imp représente un composé d'imprégnation basique constitué d'un métal alcalin ou alcalino-terreux, ou de mélanges de plusieurs de ces métaux, associé à un contre-anion pour assurer la neutralité électrique,
- n représente 1, 2 ou 3,
- h représente 0, 1 ou 2,
- p représente un nombre compris entre 0 et 1/3 et correspond à un rapport molaire entre l'imprégnant Imp et l'imprégné $(PO_4)_n \, H_h \, M$.

Parmi les aminonitriles de formule (II) les plus importants sont ceux qui conduisent aux lactames servant de matière première pour la préparation des polyamides 4, 5, 6 et 10, c'est-à-dire ceux dans la formule desquels le symbole R représente un radical alkylène linéaire ayant 2, 3, 4 ou 8 atomes de carbone. Le composé de formule (II) préféré est l'amino-6 capronitrile (ou epsilon-capronitrile), qui conduit au caprolactame dont la polymérisation fournit le polyamide 6.

Parmi les métaux des groupes 2a, 3b, 4b ,5b, 6b, 7b, 8, 2b, 3a, 4a et 5a de la classification périodique des éléments, on peut citer notamment le béryllium, le magnésium, le calcium, le strontium, le baryum, l'aluminium, le bore, le gallium, l'indium, l'yttrium, les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium, le zirconium, le titane, le vanadium, le niobium, le fer, le germanium, l'étain, le bismuth.

Parmi les phosphates de lanthanides, on peut distinguer une première famille qui regroupe les orhophosphates de terres rares légères, également dénommées terres rares cériques, incluant le lanthane, le cérium, le praséodyme, le néodyme, le samarium et l'europium. Ces orthophosphates sont dimorphiques. Ils présentent une structure hexagonale et évoluent vers une structure monoclinique, lorsqu'ils sont chauffés à une température de 600 à 800°C.

Une deuxième famille de phosphates de lanthanides regroupe les orthophosphates de gadolinium, de terbium et de dysprosium. Ces orthophosphates présentent la même structure que les orthophosphates de terres rares cériques, mais présentent en plus une troisième phase cristalline de structure quadratique à haute température (vers 1700°C).

Une troisième famille de phosphates de lanthanides regroupe les orthophosphates de terres rares lourdes, appelées également terres rares yttriques, incluant l'yttrium, l'holmium, l'erbium, le thulium, l'ytterbium et le lutécium. Ces composés cristallisent uniquement sous la forme quadratique.

Parmi les différentes familles d'orthophosphates de terres rares précitées, on fait appel préférentiellement aux orthophosphate de terres rares cériques.

On peut mettre en oeuvre des phosphates métalliques de formule (II) qui sont des mélanges de phosphates de plusieurs des métaux indiqués précédemment ou des phosphates mixtes de plusieurs des métaux indiqués précédemment ou encore des phosphates mixtes contenant un ou plusieurs des métaux indiqués précédemment et un ou plusieurs autres métaux tels que les métaux alcalins ou alcalino-terreux.

Les contre-anions entrant dans la formule du composé d'imprégnation Imp sont basiques. On peut notamment utiliser les ions hydroxyde, phosphate, hydrogénophosphate, dihydrogénophosphate, chlorure, fluorure, nitrate, benzoate, oxalate, sans que ces citations soient limitatives.

Le rapport molaire p est de préférence compris entre 0,02 et 0,2.

Si l'on se réfère aux techniques générales de préparation de phosphates (telles que décrites notamment dans "PASCAL P. Nouveau traité de chimie minérale" tome X (1956), pages 821-823 et dans "GMELINS Handbuch der anorganischen Chemie" (8$^{ème}$ édition) volume 16 (C), pages 202-206 (1965), on peut distinguer deux voies principales d'accès aux phosphates. D'une part, la précipitation d'un sel soluble du métal (chlorure, nitrate) par l'hydrogénophosphate d'ammonium ou l'acide phosphorique.D'autre part, la dissolution de l'oxyde ou du carbonate du métal (insolubles) avec de l'acide phosphorique, généralement à chaud, suivie d'une reprécipitation.

Les phosphates précipités obtenus selon l'une des voies indiquées peut être séché, traité par une base organique (telle que l'ammoniaque) ou minérale (telle qu'un hydroxyde de métal alcalin) et être soumis à une calcination, ces trois opérations pouvant être réalisées dans l'ordre indiqué ou dans un ordre différent.

Les phosphates métalliques de formule (III) pour lesquels le symbole p est supérieur à 0, peuvent être préparés par imprégnation du composé $(PO_4)_n H_h M$ préparé selon l'une des techniques décrites précédemment, avec une solution ou une suspension de Imp dans un solvant volatil, tel que l'eau de préférence.

Les résultats sont d'autant meilleurs que Imp est plus soluble et que le composé $(PO_4)_n H_h M$ est plus fraîchement fabriqué.

Ainsi un procédé avantageux de préparation des phosphates de formule (II) consiste :

a) à réaliser la synthèse du composé $(PO_4)_n H_h M$ ; puis de préférence sans séparer $(PO_4)_n H_h M$ du milieu réactiannel ;
b) à introduire l'imprégnant Imp dans le milieu réactionnel ;
c) à séparer l'éventuel liquide résiduel d'avec le solide réactionnel ;
d) à sécher et éventuellement à calciner.

Les performances du catalyseur de formule (III) et notamment sa résistance à la désactivation peuvent être encore améliorées par une calcination. La température de calcination sera avantageusement comprise entre 300°C et 1000°C et de préférence entre 400°C et 900°C. La durée de la calcination peut varier dans de larges limites. A titre indicatif, elle se situe généralement entre 1 heure et 24 heures.

Parmi les catalyseurs de formule (III) préférés dans le procédé de l'invention, on peut citer plus particulièrement le phosphate de lanthane, le phosphate de lanthane calciné, le phosphate de lanthane associé à un dérivé du césium, du rubidium ou du potassium, le phosphate de cérium calciné, le phosphate de cérium associé à un composé du césium, du rubidium ou du potassium, le phosphate de samarium associé à un composé du césium, du rubidium ou du potassium, le phosphate d'aluminium, le phosphate d'aluminium associé à un composé du césium, du rubidium ou du potassium, le phosphate de niobium calciné, le phosphate de niobium associé à un composé du césium, du rubidium ou du potassium, l'hydrogénophosphate de zirconium calciné, l'hydrogénophosphate de zirconium associé à un composé du césium, du rubidium ou du potassium.

Les oxydes massiques acides pouvant servir comme catalyseurs solides dans l'étape d'hydrolyse cyclisante sont notamment des oxydes métalliques, des mélanges d'oxydes métalliques ou des oxydes métalliques modifiés pour les rendre acides, notamment par action d'un dihalogène, d'un halogénure d'ammonium ou d'un acide comme l'acide sulfurique ou les acides halohydriques. L'halogène ainsi éventuellement introduit pour acidifier l'oxyde massique est de préférence le chlore ou le fluor.

Les oxydes massiques amphotères sont les oxydes à caractère amphotère ou ceux qui sont rendus amphotères par leur mode de préparation ou par un traitement ultérieur.

A titre d'exemples non limitatifs d'oxydes massiques acides ou amphotères, on peut citer les mélanges $SiO_2/Al_2O_3$, $SiO_2/Ga_2O_3$, $SiO_2/Fe_2O_3$, $SiO_2/B_2O_3$, les alumines halogénées telles que notamment les alumines chlorées et les alumines fluorées, la zircone sulfatée, l'oxyde de niobium, l'oxyde de tungstène, la thorine, la zircone, le dioxyde de titane, la cérine, les silices, les alumines.

Parmi ces oxydes massiques, les alumines qui peuvent être utilisées comme catalyseur solide dans l'étape d'hydrolyse cyclisante sont de structure très variée. Cependant, il est préférable de choisir parmi les différentes alumines actives pour cette réaction, celles qui se désactivent le moins. C'est pourquoi l'on choisit de préférence les alumines ayant une surface spécifique mesurée par la méthode BET supérieure ou égale à 5 m$^2$/g et encore plus préférentiellement supérieure ou égale à 10 m$^2$/g.

De préférence l'alumine mise en oeuvre dans le procédé de l'invention a une surface spécifique égale ou inférieure à 500 m$^2$/g.

Les alumines qui peuvent être utilisées dans le présent procédé sont tout d'abord les alumines ayant une surface spécifique supérieure ou égale à 10 m$^2$/g et inférieure ou égale à 280 m$^2$/g ainsi qu'un volume des pores de diamètre supérieur à 500 angstroms supérieur ou égal à 10 ml/100 g.

La surface spécifique BET est la surface spécifique déterminée par adsorption d'azote conformément à la norme ASTM D 3663-78 établie à partir de la méthode BRUNAUER-EMMETT-TELLER décrite dans le périodique "The Journal of the American Society", 60, 309 (1938).

Le volume des pores de diamètre supérieur à 500 Å représente le volume cumulé créé par tous les pores de taille supérieure à un diamètre de 500 Å. Ce volume est mesuré par la technique de la pénétration du mercure, dans laquelle on applique la loi de Kelvin.

De préférence, les alumines de cette première famille présentent un volume des pores de diamètre supérieur à 500 Å supérieur ou égal à 20 ml/100 g et encore plus préférentiellement supérieur ou égal à 30 ml/100 g.

De préférence également les alumines de cette première famille présentent une surface spécifique supérieure ou égale à 50 m$^2$/g.

Les alumines qui peuvent être utilisées dans le présent procédé sont aussi les alumines ayant une surface spécifique supérieure ou égale à 50 m$^2$/g et inférieure ou égale à 280 m$^2$/g ainsi qu'un volume des pores de diamètre supérieur à 70 angstroms supérieur ou égal à 30 ml/100 g.

De préférence, les alumines de cette deuxième famille présentent un volume des pores de diamètre supérieur à 70 Å supérieur ou égal à 45 ml/100 g.

De préférence également les alumines de cette deuxième famille présentent une surface spécifique supérieure ou égale à 80 m$^2$/g.

Les alumines qui peuvent être utilisées dans le présent procédé sont également les alumines ayant une surface spécifique supérieure ou égale à 280 m$^2$/g ainsi qu'un volume poreux total supérieur ou égal à 15 ml/100 g.

De préférence, les alumines de cette troisième famille présentent un volume poreux total supérieur ou égal à 22 ml/100 g et encore plus préférentiellement supérieur ou égal à 30 ml/100 g.

Les alumines sont aussi caractérisées par leur acidité.

Cette acidité peut être mesurée par le test d'isomérisation du butène-1 en butène-2.

Ce test est basé sur la réaction d'isomérisation du butène-1 en un mélange de cis-butène-2 et trans-butène-2 à une température T (T = 400°C dans le cas présent).

La réaction d'isomérisation est un équilibre thermodynamique. On peut définir deux constantes :

- la constante d'équilibre théorique Kth(T) déterminée par le calcul :

$$kth(T) = \frac{\text{[cis-butène-2]eq} + \text{[trans-butène-2]eq}}{\text{[butène-1]eq} + \text{[cis-butène-2]eq} + \text{[trans-butène-2]eq}}$$

où [butène]eq représente la concentration de chacun des isomères en équilibre à la température T ;

- la constante d'équilibre réelle K(T) déterminée par le résultat des mesures :

$$K(T) = \frac{\text{[cis-butène-2]} + \text{[trans-butène-2]}}{\text{[butène-1]} + \text{[cis-butène-2]} + \text{[trans-butène-2]}}$$

où [butène] représente la concentration de chacun des isomères en sortie du réacteur à la température T.

Le pouvoir isomérisant A des alumines est défini par l'activité par rapport à l'équilibre :

$$A(T) = \frac{K(T)}{Kth(T)} \times 100$$

En pratique le test est réalisé dans un réacteur phase vapeur fonctionnant en mode pulsé, dans lequel on introduit 500 mg d'alumine broyée (particules comprises entre 400 et 500 $\mu$m). L'alumine est conditionnée pendant 2 heures à 250°C sous courant d'hélium avec un débit de 2,5 litre/heure. Puis l'alumine est portée à une température de 400°C et on injecte en amont de celle-ci 1 millilitre de butène-1 dans le flux d'hélium. L'analyse des gaz de sortie est réalisée par chromatographie en phase gazeuse et permet de mesurer les quantités de butène-1 et de butène-2 cis et trans récupérées.

Ce pouvoir isomérisant A est corrigé du pouvoir isomérisant obtenu dans les mêmes conditions avec le réacteur vide. Le pouvoir isomérisant corrigé $A_c$ représente l'acidité desdites alumines

Lorsque la teneur en métal alcalin ou alcalino-terreux présent dans l'alumine est inférieure à 60 mmol par 100 g d'alumine, plus la valeur de $A_c$ est élevée, plus l'alumine est acide.

Généralement les alumines sont obtenues par déhydratation de gibbsite, de bayerite, de nordstrandite ou de leurs différents mélanges. On peut se référer par exemple à l'encyclopédie KIRK-OTHMER, volume 2, pages 291-297.

On peut préparer les alumines mises en oeuvre dans le présent procédé par mise en contact d'une alumine hydratée, sous forme finement divisée, avec un courant de gaz chaud à une température comprise entre 400°C et 1000°C, puis maintien du contact entre l'hydrate et les gaz pendant une durée allant d'une fraction de seconde jusqu'à 10 secondes et enfin séparation de l'alumine partiellement déshydratée et des gaz chauds. On peut notamment se référer au procédé décrit dans le brevet américain US 2 915 365.

On peut également procéder à l'autoclavage d'agglomérés des alumines obtenues précédemment, en milieu aqueux, éventuellement en présence d'acide, à une température supérieure à 100°C et de préférence comprise entre 150°C et 250°C, pendant une durée de préférence comprise entre 1 et 20 heures, puis à leur séchage et leur calcination.

La température de calcination est réglée de telle façon que l'on obtienne des surfaces spécifiques et des volumes poreux situés dans les zones de valeurs indiquées précédemment.

En raison de leurs principaux procédés de fabrication, les alumines mises en oeuvre dans le présent procédé contiennent le plus souvent du sodium, dont la teneur est habituellement exprimée en poids de $Na_2O$ par rapport au poids de l'alumine.

Le catalyseur peut être mis en oeuvre sous diverses formes telles que poudre, billes, concassés, extrudés, pastilles, la mise en forme pouvant éventuellement être réalisée à l'aide d'un liant.

Il peut s'agir tout d'abord de billes d'alumine issues d'une mise en forme par oil-drop (ou coagulation en gouttes). Ce type de billes peut par exemple être préparé par un procédé selon l'enseignement des brevets EP-A-0 015 801 ou EP-A-0 097 539. Le contrôle de la porosité peut être réalisé en particulier, selon le procédé décrit dans le brevet EP-A-0 097 539, par coagulation en gouttes d'une suspension ou d'une dispersion aqueuse d'alumine ou d'une solution d'un sel basique d'aluminium se présentant sous la forme d'une émulsion constituée d'une phase organique, d'une phase aqueuse et d'un agent de surface ou d'un émulsionnant. Ladite phase organique peut en particulier être un hydrocarbure.

Il peut également s'agir de concassés d'alumine. Ces concassés peuvent être issus du concassage de tout type de matière à base d'alumine telle que, par exemple, des billes obtenues par tous types de procédé (oil-drop, drageoir ou tambour toumant) ou des extrudés. Le contrôle de la porosité de ces concassés se fait par le choix de la matière à base d'alumine que l'on concasse pour les obtenir.

Il peut aussi s'agir d'extrudés alumine. Ceux-ci peuvent être obtenus par malaxage, puis extrusion d'une matière à base d'alumine, ladite matière pouvant être issue de la déshydratation rapide d'hydrargillite ou de la précipitation d'un gel d'alumine. Le contrôle de la porosité de ces extrudés peut être réalisé par le choix de l'alumine mise en oeuvre et par les conditions de préparation de cette alumine ou par les conditions de malaxage de cette alumine avant extrusion. L'alumine peut ainsi être mélangée lors du malaxage à des porogènes. A titre d'exemple, les extrudés peuvent être préparés par le procédé décrit dans le brevet US 3 856 708.

Il peut dans certain cas être avantageux qu'au moins une partie du volume libre du réacteur soit occupé par un solide inerte, tel que par exemple du quartz, afin de favoriser la vaporisation et la dispersion des réactifs.

Comme pour les alumines, de manière générale le catalyseur solide de l'étape d'hydrolyse cyclisante est mis en oeuvre sous forme de poudre, de pastilles, de concassés, de billes ou d'extrudés, ladite mise en forme pouvant éventuellement être réalisée à l'aide d'un liant. Il peut dans certain cas être avantageux qu'au moins une partie du volume libre du réacteur soit occupé par un solide inerte, tel que par exemple du quartz, afin de favoriser la vaporisation et la dispersion des réactifs.

La réaction d'hydrolyse cyclisante nécessite la présence d'eau. Le rapport molaire entre l'eau et l'aminonitrile engagés se situe habituellement entre 0,5 et 50 et de préférence entre 1 et 20. La valeur supérieure de ce rapport n'est pas critique pour l'invention, mais des rapports plus élevés n'ont guère d'intérêt pour des questions économiques.

L'aminonitrile et l'eau peuvent être engagés sous forme de leurs mélanges à l'état de vapeurs ou être introduits séparément dans le réacteur. On peut réaliser une prévaporisation des réactifs qui circulent ensuite dans une chambre de mélange.

On peut sans inconvénient utiliser tout gaz inerte comme vecteur, tel que l'azote, l'hélium ou l'argon.

La température à laquelle est mise en oeuvre l'étape d'hydrolyse cyclisante doit être suffisante pour que les réactifs soient bien à l'état de vapeurs. Elle se situe généralement entre 200°C et 450°C et de préférence entre 250°C et 400°C.

Le temps de contact entre l'aminonitrile et le catalyseur n'est pas critique. Il peut varier selon l'appareillage utilisé notamment. Ce temps de contact se situe de préférence entre 0,5 à 200 secondes et encore plus préférentiellement entre 1 et 100 secondes.

La pression n'est pas un paramètre critique de cette étape du procédé. Ainsi on peut opérer sous des pressions

de 10$^{-3}$ bar à 200 bar. De préférence, on mettra en oeuvre le procédé sous une pression de 0,1 à 20 bar.

Il n'est pas exclu d'utiliser un solvant inerte dans les conditions réactionnelles, tel que par exemple un alcane, un cycloalcane, un hydrocarbure aromatique ou l'un de ces hydrocarbures précédents halogéné, et d'avoir ainsi une phase liquide dans le flux réactionnel.

Le lactame obtenu par le procédé selon l'invention peut être purifié, généralement par distillation, avant d'être polymérisé. Cette purification est beaucoup plus aisée au stade du lactame qu'à celui de l'aminonitrile. Il s'avère d'ailleurs que les sous-produits contenus dans l'aminonitrile engagé à l'étape d'hydrolyse cyclisante sont pour une part notable décomposés au cours de cette étape et sont alors facilement éliminés.

La distillation du lactame peut être en particulier effectuée en présence d'une base forte, telle qu'un hydroxyde de métal alcalin, la présence de cette base facilitant la séparation du lactame des sous-produits et de l'aminonitrile qui n'aurait pas été transformé. La soude ou la potasse conviennent notamment bien.

Les exemples qui suivent illustrent l'invention.

EXEMPLE 1

Dans un réacteur en acier inoxydable de 3,6 l, équipé d'une agitation de type rushtone cavitator, de moyens d'introduction des réactifs et de l'hydrogène et d'un système de régulation de température, on charge :

- adiponitrile         1710 g

- hexaméthylène diamine         574,4 g

- eau         253 g

- KOH         0,63 g

- Ni de Raney (à 1,7 % de Cr)         30,3 g de Ni

Dans cet exemple il y a 0,4 mol KOH/kg Ni.

On chauffe le mélange réactionnel à 50°C après avoir purgé le réacteur à l'azote, puis à l'hydrogène ; la pression est alors réglée à 2 MPa à cette température par addition continue d'hydrogène. L'avancement de la réaction est suivi par la consommation d'hydrogène. Lorsque 2,2 équivalents d'hydrogène par rapport à l'adiponitrile chargé ont été consommés, on arrête la réaction par arrêt de l'agitation et refroidissement du mélange réactionnel. On dose par chromatographie en phase vapeur l'adiponitrile (ADN) restant et l'amino-6 capronitrile (ACN) formé et l'on calcule le taux de transformation (TT) de l'ADN et le rendement en ACN par rapport à l'ADN transformé (RT).

On obtient les résultats suivants :

- durée de la réaction:         118 min
- TT de l'ADN :         81 %
- RT en ACN :         60,3 %

Cette opération est répétée 7 fois et l'ensemble des masses réactionnelles, après séparation du catalyseur par filtration, est regroupé pour être distillé. L'aminocapronitrile est distillé à 120°C sous 2660 Pa, à l'aide d'une colonne de 70 mm de diamètre, à garnissage en acier inoxydable, ayant une efficacité correspondant à 30 plateaux théoriques.

L'aminocapronitrile ainsi obtenu contient:

- moins de 0,01 % en poids d'adiponitrile
- 0,1 % en poids d'hexaméthylènediamine
- 98,4 % en poids d'amino-6 capronitrile
- 1,5 % en poids de diverses amines et imines dont la principale est le (amino-6' hexaméthylene-imino)-6 hexanenitrile.

L'étape d'hydrolyse cyclisante de l'amino-6 capronitrile (ACN) ainsi préparé est réalisée dans un réacteur cylindrique de 20 ml en verre Pyrex, disposé verticalement et muni de moyens de chauffage, d'ouvertures pour l'arrivée et la sortie des flux gazeux et d'un système d'injection des réactifs.

On charge successivement dans ce réacteur 10 ml de quartz, 1 ml d'alumine sous forme de poudre de granulométrie 400-500 micromètres et à nouveau 10 ml de quartz.

Le réacteur ainsi chargé est chauffé à 400°C sous courant d'air (avec un débit de 1,5 litre/heure) pendant 2 heures. Ensuite le réacteur est refroidi à 320°C ( température de réaction choisie) et mis sous courant d'azote (débit de 17,6 ml/min).

On injecte alors, à l'aide d'une pompe, un mélange d'ACN et d'eau (rapport pondéral 50/50, soit un rapport molaire eau/ACN de 6,2). Le débit d'injection du mélange est de 1,14 g/h.

A la sortie du réacteur, les vapeurs sont condensées dans un piège en verre à température ambiante.

Le mélange réactionnel final est dosé en chromatographie en phase gazeuse.

On détermine le taux de transformation (TT) de l'aminocapronitrile, le rendement (RT) en caprolactame (CPL) par rapport à l'aminocapronitrile transformé;

Par diverses techniques d'analyse (chromatographie en phase gazeuse couplée spectrographie de masse, résonance magnétique nucléaire notamment), on constate la disparition complète des sous-produits amines et imines présents dans l'aminocapronitrile mis en oeuvre.

On obtient les performances suivantes stables pour une durée de réaction de 50 heures :

- TT de l'ACN:  $\geq 99\%$
- RT en CPL:  $\geq 98\%$

EXEMPLE 2

Dans un réacteur métallique, équipé d'une agitation de type cavitator, de moyens d'introduction des réactifs et de l'hydrogène et de différents systèmes de régulation, on introduit avec les charges suivantes :

- adiponitrile     2856 kg

- hexaméthylène diamine     1151 kg

- eau     588 kg

- KOH     0,83 kg

- Ni de Raney (à 1,7 % de Cr)     37 kg

Dans cet exemple il y a 0,4 mol KOH/kg Ni.

On opère dans les conditions décrites pour l'exemple 1.

On obtient les résultats suivants :

- durée de la réaction :     3 h 30
- TT de l'ADN:     86 %
- RT en ACN :     64 %

Après séparation du catalyseur par filtration, environ 6 kg de mélange réactionnel sont distillés à 120°C sous 2660 Pa, à l'aide d'une colonne de 75 mm de diamètre, à gamissage en acier inoxydable, ayant une efficacité correspondant à 11 plateaux théoriques.

Pour la deuxième étape du procédé, on utilise une fraction de la distillation précédente qui contient :

- 0,02 % en poids d'adiponitrile
- 0,36 % en poids d'hexaméthylènediamine
- 97,1 % en poids d'amino-6 capronitrile
- 2,5 % en poids de diverses amines et imines dont la principale est le (amino-6' hexaméthylène-imino)-6 hexanenitrile.

Afin de mieux démontrer que le procédé de l'invention permet de mettre en oeuvre dans l'étape d'hydrolyse cyclisante, un amino-6 capronitrile relativement peu purifié, on a rajouté à cette fraction d'aminocapronitrile (ACN) distillé différentes impuretés supplémentaires. On obtient ainsi un ACN contenant :

- 1 % en poids d'adiponitrile
- 1 % en poids d'hexaméthylènediamine
- 2,5 % en poids de diverses amines et imines dont la principale est le (amino-6' hexaméthylène-imino)-6 hexanenitrile

- 1 % en poids d'imino-1 cyano-2 cyclopentane
- 1 % en poids d'hexaméthylène-imine (HMI)
- 1 % en poids de bis(hexaméthylènetriamine) (BHT)
- 92,5 % en poids d'amino-6 capronitrile.

L'étape d'hydrolyse cyclisante de l'ACN ainsi préparé est réalisée dans un réacteur cylindrique de 20 ml en verre Pyrex, disposé verticalement et muni de moyens de chauffage, d'ouvertures pour l'arrivée et la sortie des flux gazeux et d'un système d'injection des réactifs.

On charge successivement dans ce réacteur 3 ml de quartz, 2 ml (0,87 g) d'alumine (de surface spécifique BET de 130 m$^2$/g) sous forme de poudre de granulométrie 300-600 micromètres et à nouveau 5 ml de quartz.

Le réacteur ainsi chargé est chauffé à 400°C sous courant d'air (avec un débit de 1,5 litre/heure) pendant 2 heures. Ensuite le réacteur est refroidi à 320°C ( température de réaction choisie) et mis sous courant d'azote (débit de 88 ml/min).

On injecte alors, à l'aide d'une pompe, un mélange d'ACN et d'eau (rapport molaire eau/ACN de 1,1). Le débit d'injection du mélange est de 11 g/h.

A la sortie du réacteur, les vapeurs sont condensées dans un piège en verre à température ambiante.

Le mélange réactionnel final est dosé en chromatographie en phase gazeuse.

On détermine le taux de transformation (TT) de l'aminocapronitrile, le rendement (RT) en caprolactame (CPL) par rapport à l'aminocapronitrile transformé;

Par diverses techniques d'analyse (chromatographie en phase gazeuse couplée spectrographie de masse, résonance magnétique nucléaire notamment), on constate la disparition complète des sous-produits amines et imines présents dans l'aminocapronitrile mis en oeuvre.

On obtient les performances suivantes stables pour une durée de réaction de 7 heures :

- TT de l'ACN:     63 %
- RT en CPL:     100%

## Revendications

1. Procédé de préparation de lactame, **caractérisé en ce qu'**il comprend les étapes successives suivantes:

   - hémihydrogénation en aminonitrile par l'hydrogène, en présence d'un catalyseur et d'une base minérale forte, d'un dinitrile aliphatique de formule générale (1) :

   $$NC\text{-}R\text{-}CN \qquad\qquad (1)$$

   dans laquelle R représente un groupement alkylène ou alcénylène, linéaire ou ramifié, ayant de 1 à 12 atomes de carbone et de préférence de 1 à 6 atomes de carbone,
   - extraction de l'aminonitrile du milieu réactionnel par distillation pour récupérer un aminonitrile présentant une teneur en dinitrile variant de 0,005% à 5 % en poids et une teneur en sous-produits à fonction imine ou amine variant de 0,2 à 5 % en poids,
   - hydrolyser l'aminonitrile distillé en phase vapeur avec de l'eau en présence ou non d'un catalyseur pour produire un lactame.

2. Procédé selon la revendication 1, **caractérisé en ce que** l'hémihydrogénation du dinitrile en aminonitrile correspondant, à l'aide d'hydrogène, est effectuée en présence d'un catalyseur à base de nickel de Raney, de cobalt de Raney, de nickel de Raney ou de cobalt de Raney comportant un élément dopant choisi parmi les éléments des groupes IVb, VIb, VIIb et VIII de la classification périodique des éléments et d'une base minérale forte dérivant d'un métal alcalin ou alcalino-terreux.

3. Procédé selon la revendication 2, **caractérisé en ce que** la base minérale forte est choisie parmi les hydroxydes, les carbonates et les alcanolates de métal alcalin ou de métal alcalino-terreux et de préférence parmi les hydroxydes, les carbonates et les alcanolates de métal alcalin.

4. Procédé selon la revendication 2, **caractérisé en ce que** la quantité de catalyseur mise en oeuvre est de 0,5 % à 50 % en poids de catalyseur par rapport au poids total du milieu réactionnel

**5.** Procédé selon l'une des revendications 1 à 4, **caractérisé en ce que** l'étape d'hémihydrogénation est effectuée à une température inférieure ou égale à 150°C.

**6.** Procédé selon l'une des revendications 1 à 5, **caractérisé en ce que** l'étape d'hémihydrogénation est effectué sous une pression en hydrogénecomprise entre 1 bar (0, 10 MPa) et 100 bar (10 MPa).

**7.** Procédé selon l'une des revendications précédentes, **caractérisé en ce que** le catalyseur solide utilisé dans la réaction d'hydrolyse est choisi dans le groupe comprenant un phosphate métallique, un oxyde massique acide ou amphotère, les tamis moléculaires, tels qu'une zéolithe acide, une silicalite, un tamis moléculaire non-zéolithique .

**8.** Procédé selon la revendication 7, **caractérisé en ce que** le phosphate métallique est un phosphate métallique de formule générale (III) :

$$(PO_4)_n \ H_h \ M, \ (Imp)_p \qquad\qquad (III)$$

dans laquelle :

- M représente un élément divalent, trivalent, tétravalent ou pentavalent choisi dans les groupes 2a, 3b, 4b 5b, 6b, 7b, 8, 2b, 3a, 4a et 5a de la classification périodique des éléments ou un mélange de plusieurs de ces éléments ou M=O lorsque M représente certains éléments pentavalents,
- Imp représente un composé d'imprégnation basique constitué d'un métal alcalin ou alcalino-terreux, ou de mélanges de plusieurs de ces métaux, associé à un contre-anion pour assurer la neutralité électrique
- n représente 1, 2 ou 3,
- h représente 0, 1 ou 2,
- p représente un nombre compris entre 0 et 1/3 et correspond à un rapport molaire entre l'imprégnant Imp et l'imprégné $(PO_4)_n \ H_h \ M$.

**9.** Procédé selon la revendication 7, **caractérisé en ce que** l'oxyde massique est une alumine activée.

**10.** Procédé selon la revendication 9, **caractérisé en ce que** l'alumine activée a une surface spécifique comprise entre 5 $m^2$/g et 500 $m^2$/g.

**11.** Procédé selon l'une des revendications, précédentes, **caractérisé en ce que** dans l'étape d'hydrolyse, le rapport molaire entre l'eau et l'aminonitrile est compris entre 0,5 et 50.

**12.** Procédé selon l'une des revendications précédentes, **caractérisé en ce que** la température à laquelle est mise en oeuvre l'étape d'hydrolyse cyclisante est comprise entre 200°C et 450°C.

**Patentansprüche**

**1.** Verfahren zur Herstellung von Lactam, **dadurch gekennzeichnet, daß** es die folgenden aufeinanderfolgenden Stufen umfaßt:

- Hemihydrierung zum Aminonitril durch Wasserstoff in Anwesenheit eines Katalysators und einer starken Mineralbase eines aliphatischen Dinitrils der allgemeinen formel (I):

$$NC-R-CN \qquad\qquad (I)$$

worin R eine lineare oder verzweigte Alkylen- oder Alkenylengruppe mit 1 bis 12 Kohlenstoffatomen, und vorzugsweise 1 bis 6 Kohlenstoffatomen, bedeutet;
- Extraktion des Aminonitrils des Reaktionsmilieus durch Destillation zur Gewinnung eines Aminonitrils mit einem Gehalt an Dinitril von 0,005 bis 5 Gew.-% einzusetzen und einem Gehalt an Nebenproduckten mit Imin- oder Aminfunktion von 0,2 bis 5 Gew.-% einzusetzen
- Hydrolyse des destillierten Aminonitrils in Dampfphase mit Wasser in Gegenwart oder Abwesenheit eines Katalysators, um ein Lactam zu bilden.

2. Verfahren gemäß Anspruch 1, **dadurch gekennzeichnet, daß** die Hemihydrierung des Dinitrils zum entsprechenden Aminonitril mit Wasserstoff in Anwesenheit eines Katalysators auf Basis von Raney-Nickel, Raney-Kobalt, Raney-Nickel oder Raney-Kobalt, umfassend ein Dotierungselement, ausgewählt unter den Elementen der Gruppen IVb, VIb, VIIb und VIII des Periodensystems der Elemente, und einer starken mineralischen Base, die sich von einem Alkali oder Erdalkalimetall ableitet, durchgeführt wird.

3. Verfahren gemäß Anspruch 2, **dadurch gekennzeichnet, daß** die starke mineralische Base unter den Hydroxiden, den Carbonaten und den Alkanolaten von Alkalimetall oder Erdalkalimetall, und vorzugsweise unter den Hydroxiden, den Carbonaten und den Alkanolaten von Alkalimetall, durchgeführt wird.

4. Verfahren gemäß Anspruch 2, **dadurch gekennzeichnet, daß** die Menge des eingesetzten Katalysators 0,5 bis 50%, ausgedrückt als Gewicht des Katalysators in Bezug auf das Gesamtgewicht des Reaktionsmilieus, beträgt.

5. Verfahren gemäß einem der Ansprüche 1 bis 4, **dadurch gekennzeichnet, daß** die Stufe der Hemihydrierung bei einer Temperatur von niedriger als oder gleich 150°C durchgeführt wird.

6. Verfahren gemäß einem der Ansprüche 1 bis 5, **dadurch gekennzeichnet, daß** die Stufe der Hemihydrierung unter einem Wasserstoffdruck zwischen 1 bar (0,10 MPa) und 100 bar (10 MPa) durchgeführt wird.

7. Verfahren gemäß einem der vorhergehenden Ansprüche, **dadurch gekennzeichnet, daß** der bei der Hydrolysereaktion verwendete feste Katalysator ausgewählt wird aus der Gruppe, umfassend ein Metallphosphat, ein saures oder amphoteres voluminöses Oxid, Molekularsiebe, ein saures Zeolith, ein Silikalit, ein nicht-zeolithisches Molekularsieb.

8. Verfahren gemäß Anspruch 7, **dadurch gekennzeichnet, daß** das Metallphosphat ein Metallphosphat der allgemeinen Formel (III):

$$(PO_4)_n \, H_h \, M, \, (Imp)_p \qquad\qquad (III)$$

ist, worin:

- M ein zweiwertiges, dreiwertiges, vierwertiges oder fünfwertiges Element, ausgewählt unter den Gruppen 2a, 3b, 4b, 5b, 6b, 7b, 8, 2b, 3a, 4a und 5a des Periodensystems der Elemente und einem Gemisch von mehreren dieser Elemente, bedeutet, oder M = O wenn M bestimmte fünfwertige Elemente wiedergibt;
- Imp eine aus einem Alkali oder Erdalkalimetall oder Gemischen mehrerer dieser Metalle bestehende basische Imprägnierungsverbindung, assoziiert an ein Gegenanion zur Sicherstellung der elektrischen Neutralität, wiedergibt;
- n für 1, 2 oder 3 steht;
- h für 0, 1 oder 2 steht;
- p für eine Zahl zwischen 0 und 1/3 steht und dem Molverhältnis zwischen Imprägnierungsmittel Imp und der imprägnierten Komponente $(PO_4)_n \, H_h \, M$ entspricht.

9. Verfahren gemäß Anspruch 7, **dadurch gekennzeichnet, daß** das voluminöse Oxid aktiviertes Aluminiumoxid ist.

10. Verfahren gemäß Anspruch 9, **dadurch gekennzeichnet, daß** das aktivierte Aluminiumoxid eine spezifische Oberfläche zwischen 5 $m^2/g$ und 500 $m^2/g$ besitzt.

11. Verfahren gemäß einem der vorhergehenden Ansprüche, **dadurch gekennzeichnet, daß** bei der Hydrolysestufe das Molverhältnis zwischen Wasser und Aminonitril zwischen 0,5 und 50 beträgt.

12. Verfahren gemäß einem der vorhergehenden Ansprüche, **dadurch gekennzeichnet, daß** die Temperatur, bei der die Stufe der zyklisierenden Hydrolyse durchgeführt wird, zwischen 200 und 450 °C, liegt.

**Claims**

1. Process for the preparation, of a lactam, **characterized in that** it comprises the following successive steps:

   - semihydrogenation into aminonitrile by hydrogen, in the presence of a catalyst and a strong mineral base, of an aliphatic dinitrile of general formula (1):

$$NC-R-CN \qquad\qquad (1)$$

   in which R represents a linear or branched alkylene or alkenylene group having from 1 to 12 carbon atoms and preferably from 1 to 6 carbon atoms,
   - extraction of the aminonitrile from the reaction mixture by distillation in order to recover an aminonitrile having a dinitrile content from 0,005% to 5% by weight and a content of by-products with an amine or imine functional group from 0,2 to 5% by weight
   - hydrolysis of the disilled aminonitrile in the vapour phase with water, in the presence or absence of a catalyst for producing a lactam.

2. Process according to Claim 1, **characterized in that** the semihydrogenation of the dinitrile to the corresponding aminonitrile, with the aid of hydrogen, is performed in the presence of a catalyst based on Raney nickel, Raney cobalt, Raney nickel or Raney cobalt containing a dopant element chosen from the elements of groups IVb, VIb, VIIb and VIII of the Periodic Table of the Elements and of a strong inorganic base derived from an alkali metal or alkaline-earth metal.

3. Process according to Claim 2, **characterized in that** the strong inorganic base is chosen from alkali or alkaline-earth metal hydroxides, carbonates and alkanolates and preferably from alkali metal hydroxides, carbonates and alkanolates.

4. Process according to Claim 2, **characterized in that** the quantity of catalyst used is from 0.5% to 50% by weight of catalyst relative to the total weight of the reaction mixture.

5. Process according to one of Claims 1 to 4, **characterized in that** the semihydrogenation stage is performed at a temperature less than or equal to 150°C.

6. Process according to one of claims 1 to 5, **characterized in that** the semihydrogenation stage is performed under, a hydrogen pressure of between 1 bar (0.10 MPa) and 100 bar (10 MPa).

7. Process according to one of the preceding claims, **characterized in that** the solid catalyst used in the hydrolysis reaction is chosen from the group comprising a metal phosphate, an acidic or amphoteric bulk oxide, molecular sieves such as an acidic zeolite, a silicalite or a non-zeolite molecular sieve.

8. process according to Claim 7, **characterized in that** the metal phosphate is a metal phosphate of general formula (III):

$$MH_h(PO_4)_n(Imp)_p \qquad\qquad (III)$$

   in which:

   - M denotes a divalent, trivalent, tetravalent or pentavalent element chosen from groups 2a, 3b, 4b, 5b, 6b, 7b, 8, 2b, 3a, 4a and 5a of the Periodic Table of the Elements or a mixture of several of these elements or M=O when M denotes certain pentavalent elements,
   - Imp denotes a basic impregnating compound consisting of an alkali metal or alkaline-earth metal or of mixtures of several of these metals, used in combination with a counteranion to ensure electrical neutrality,
   - n denotes 1, 2 or 3,
   - h denotes 0, 1 or 2,
   - p denotes a number between 0 and 1/3 and corresponds to a molar ratio of the impregnating compound Imp

to the impregnated $MH_h(PO_4)_n$.

9. Process according to Claim 7, **characterized in that** the bulk oxide is an activated alumina.

10. process according to Claim 9, **characterized in that** the activated alumina has a specific surface area of between 5 $m^2$/g and 500 $m^2$/g.

11. Process according to one of the preceding claims, **characterized in that** in the hydrolysis stage the molar ratio of water to the aminonitrile is between 0.5 and 50.

12. Process according to one of the preceding claims, **characterized in that** the temperature at which the cyclizing hydrolysis stage is carried out is between 200°C and 450°C.